# FASCICULE DE BREVET EUROPEEN

(11) **EP 2 331 038 B1**
(45) Date de publication et mention de la délivrance du brevet: **11.09.2013**
(21) Numéro de dépôt: 09741339.7
(22) Date de dépôt: 24.09.2009
(51) Int. Cl.: A61F 13/02, A61F 13/12

(54) **DISPOSITIF MÉDICAL POUR SITE DE PONCTION OU DE PERFUSION**
MEDIZINISCHE VORRICHTUNG FÜR EINE PUNKTIONSSTELLE ODER INFUSIONSSTELLE
MEDICAL DEVICE FOR A PUNCTURE SITE OR INFUSION SITE

(30) Priorité: 30.09.2008 FR 0805405
(43) Date de publication de la demande: 15.06.2011
(73) Titulaire: Nephrokit, 78110 Le Vesinet (FR)
(72) Inventeur: CHAWKI, Mokhtar, F-78110 Le Vesinet (FR)
(74) Mandataire: Demulsant, Xavier
(86) Numéro de dépôt international: PCT/FR2009/001133
(87) Numéro de publication internationale: WO 2010/037922

(56) Documents cités:
- WO-A-99/08723
- WO-A-03/099143
- WO-A-2007/044647
- US-A- 5 891 074
- US-A1- 2006 155 235

## Description

L'invention se rapporte au domaine médical ou vétérinaire.

L'invention concerne plus particulièrement un dispositif de protection d'un point de ponction ou d'infusion.

Par « ponction » on désigne ici l'opération par laquelle une substance, généralement liquide, est évacuée d'une partie du corps d'un homme ou d'un animal.

Par « infusion » on désigne ici l'opération par laquelle une substance, généralement liquide, est introduite dans une cavité ou dans les vaisseaux du corps d'un homme ou d'un animal.

Par « point de ponction ou d'infusion » on désigne ici une zone de peau du corps d'un homme ou d'un animal au travers de laquelle passe un dispositif de ponction ou d'infusion (en particulier une aiguille, un cathéter, une canule).

L'invention intéresse notamment l'utilisation médicale ou vétérinaire d'un point de ponction ou d'infusion vasculaire. L'invention trouve notamment une application dans le domaine de l'hémodialyse, ou celui des perfusions à aiguilles/cathéters ou dispositifs intraveineux courts de type cathlon® (canules en PTFE).

Il est courant de retirer ou d'injecter des fluides ou d'administrer des médicaments à un patient ou un animal par l'intermédiaire d'un tube fixé à une aiguille ou un cathéter.

Un exemple de ponction est le prélèvement sanguin, soin courant permettant des examens de laboratoire sur un échantillon de sang prélevé par ponction veineuse, capillaire ou artérielle. L'aiguille hypodermique utilisée pour le prélèvement sanguin est souvent reliée à des tubes sous vide (par exemple Vacutainer®, commercialisés par la société Becton Dickinson).

Un exemple d'infusion est la perfusion intraveineuse, technique courante d'infusion parentérale permettant l'administration en goutte à goutte de médicaments, de liquides ou produits sanguins dans les veines, généralement une veine périphérique du membre supérieur. La perfusion intraveineuse permet notamment l'administration de solutés de correction de la volémie.

Pour la ponction ou l'infusion, un dispositif est inséré au travers de la peau (aiguille hypodermique, cathéter, canule).

Par exemple, les aiguilles à ailettes sont employées pour les transfusions, les prises de sang notamment. Ces aiguilles tirent leur nom de ce qu'elles sont pourvues d'une zone de pincement manuel en forme d'ailes de papillon. Le repli de ces ailettes l'une contre l'autre permet une bonne préhension et facilite l'insertion de l'aiguille sous la peau. Une fois l'aiguille mise en place, les ailettes viennent classiquement se reposer sur la peau et une bande adhésive est placée sur ces ailettes et sur la peau en vue d'éviter l'extraction de l'aiguille. Des exemples d'aiguilles à ailettes peuvent être trouvés dans les brevets américains délivrés sous les numéros suivants: 2 725 058, 3 064 648, 3 640 275, 4 194 504, 4 300 553, 4 627 842, 5 108 376, 5 149 328, 6 270 480.

Il est estimé qu'environ 80 % des patients hospitalisés font l'objet d'un traitement administré par cathéter intraveineux. Pour certains patients, les cathéters périphériques sont d'usage chronique. C'est le cas notamment des malades soufrant d'insuffisance rénale aiguë ou chronique et traités par hémodialyse, ou épuration extra rénale. La séance d'hémodialyse dure environ quatre heures, et doit être effectuée trois fois par semaine. Trois types d'accès vasculaires prédominent pour l'hémodialyse: les fistules artérioveineuses FAV, les prothèses artérioveineuses ou grafts, et les cathéters veineux centraux CVC. Les FAV sont des anastomoses créées chirurgicalement pour connecter une artère et une veine du patient, couramment dans l'avant bras, ou le bras, le plus souvent entre une artère radiale ou humérale et sa veine homonyme. Cette anastomose permet d'augmenter le débit sanguin au sein de ladite veine. La création d'une FAV modifie fortement l'aspect de l'avant bras du patient, par création de zones anévrysmales. Les aiguilles utilisées pour ponctionner les FAV sont de gros calibre, d'un diamètre interne variant typiquement entre 1.6 et 2 mm. L'aiguille amenant le sang du patient vers l'appareil de dialyse est appelée artère, celle restituant le sang vers le patient étant appelée veine.

Lorsque l'aiguille est retirée d'un vaisseau sanguin ou d'une fistule de dialyse, plusieurs risques sont à prendre en compte : risque d'hémorragie, risque de projection de sang, risque de contamination du personnel soignant, risque d'hématome, risque de contamination bactérienne.

Le risque hémorragique est très important pour certains malades souffrant d'insuffisance rénale aiguë ou chronique et traités par hémodialyse. Lors de l'hémodialyse, un anticoagulant est employé pour limiter les risques de colmatage des lumières des capillaires par thrombose. A la fin de la séance de dialyse, toute hémorragie à partir du point de ponction peut s'avérer fatale pour le patient.

Le risque de projection de sang et de contamination est important, l'expansion du SIDA et des hépatites, entre autres maladies contagieuses par voie sanguine, n'ayant fait que rendre ce risque plus redouté. Selon la circulaire de la Délégation générale de la santé, datée du 20 avril 1998 (accessible à l'adresse http://www.sante.gouv.fr), relative à la prévention de la transmission d'agents infectieux véhiculés par le sang ou les liquides biologiques lors des soins dans les établissements de santé, un accident d'exposition au sang (AES) est défini comme « *tout contact avec du sang ou un liquide biologique contenant du sang et comportant soit une effraction cutanée soit une projection sur une muqueuse (oeil) ou sur une peau lésée* ». Le risque de transmission de maladies infectieuses lors d'un AES en hémodialyse est actuellement dominé par le virus de l'hépatite C en raison de sa prévalence chez les patients hémodialysés. Le risque de transmission du VIH après exposition au sang d'un patient porteur du VIH est estimé en moyenne à 0.32%. Le risque de transmission du VHB à partir d'un patient infecté est très élevé : entre 2% et 40%. Cette forte contagiosité est liée à la quantité très importante de virus présents dans le sang et les liquides biologiques (entre 1 million à 1 milliard de particules virales par ml). Selon le rapport RAISIN (surveillance des accidents d'exposition au sang dans les établissements de santé Français en 2005, disponible à l'adresse http://www.invs.sante.fr), la majorité des accidents d'exposition du sang surviennent au moment du retrait d'une aiguille à travers la peau. Pour les séances d'hémodialyse sur FAV, le moment le plus propice au risque de contamination par projection de sang est celui de la compression, principalement au moment du retrait de l'aiguille. En effet, la compresse hémostatique peut être imparfaitement positionnée sur le point de ponction, en particulier pour des FAV présentant un fort débit ou des zones anévrysmales.

En fin de séance de dialyse, lors du débranchement, il existe un risque de contamination bactérienne, généralement staphylococcique. Ce risque est élevé au moment de la compression des points de ponction ou lors d'une récidive du saignement à distance de la séance de dialyse.

Les moyens mis en oeuvre dans les unités d'hémodialyse pour palier aux risques d'AES et d'hémorragie ne sont pas standardisés. Les protocoles de débranchement ne sont en particulier pas uniformes, plusieurs paramètres étant pris en compte : autonomie des patients, capacité physique des patients leur permettant plus ou moins de s'impliquer dans leur prise en charge, fragilité de la peau de patients pour la plupart âgés, existence ou non de zones anévrysmales, prise de médicaments anticoagulants de type acide acétylsalicylique ou Anti Vitamine K ou anti-inflammatoires. Enfin le débit de la FAV, ainsi que la présence d'hyperpression par sténose de l'abord vasculaire peuvent concourir à prolonger le temps de compression.

On connaît, dans l'art antérieur, plusieurs techniques visant à éviter les risques d'AES et d'hémorragie qui viennent d'être décrits.

Selon une technique extrêmement courante, une compresse, un tampon ou du coton est placé au point de ponction et une pression manuelle est appliquée sur cette compresse ou ce coton, une ou plusieurs bandes adhésives maintenant en place la compresse ou le coton après relâchement de la pression manuelle.

Le demandeur a constaté que cette technique courante pose plusieurs difficultés.

Chez les dialysés porteurs d'une FAV à fort débit sanguin, l'effet des anticoagulants est tel que le temps de saignement au point de ponction peut aller jusqu'à quarante cinq minutes, après la séance de dialyse (voir document WO 03/099143 page 1 lignes 11 à 20). Le maintien d'une pression sur la FAV pendant un temps aussi long est fastidieux et fatiguant. Les personnes souffrant de maladies neurodégénératives (Parkinson, syndrome parkinsonien, Alzheimer) peuvent ne pas être en mesure de maintenir une pression manuelle au point de ponction. Il en va de même chez les patients agités, ou dépressifs, épileptiques, ou atteints de chorée, ou frappés de convulsions pour diverses raisons.

Il est nécessaire de contrôler régulièrement l'arrêt du saignement au point de ponction ou d'infusion, ce qui nécessite d'enlever la compresse ou le coton. Ce contrôle peut provoquer une projection de sang, avec risque de contamination du personnel soignant, ou bien encore une hémorragie, en particulier lorsque le patient est agité ou pour les patients dialysés devant comprimer à la fois l'accès veineux et l'accès artériel de la FAV. Un saignement peut survenir lors de la compression du point de ponction, obligeant à changer de compresse, la présence de sang occultant le point de ponction. La vérification régulière de l'arrêt du saignement par retrait partiel ou total de la compresse peut entraîner l'adhésion de la compresse hémostatique ou non sur le site de ponction avec risque de décollement du clou plaquettaire.

Le document US 2004/0092999 décrit un bracelet élastique en latex sur lequel est collée une pièce hémisphérique en gomme ou latex, ce bracelet pouvant servir de garrot avant ponction veineuse, et facilitant l'hémostase après ponction, la pièce de gomme venant comprimer le point de ponction au travers d'une compresse. La pièce de gomme décrite dans ce document antérieur permet d'éviter l'application d'une pression manuelle au point de ponction. Mais l'utilisation de ce bracelet ne permet pas d'éviter les risques de projection de sang ou d'hémorragie lorsque l'on enlève la pièce de compression pour contrôler le point de ponction. Les pansements décrits dans les documents WO 99/08723, US 2005/0256438 présentent les mêmes inconvénients. Il en va de même du pansement compressif pour FAV décrit dans le document WO 03/099143.

Le document WO 2007/044647 décrit un pansement compressif délivrant un médicament. La délivrance du médicament est obtenue par rupture d'une poche frangible et passage du médicament dans une compresse recouvrant la peau du patient. En variante, la délivrance du médicament est obtenue par passage au travers d'un film non adhérant et non absorbant, perméable à l'air et à l'eau, tel qu'un film polyoléfine non tissé perforé commercialisé par la société Delstar sous la marque Delnet®.

Le document US 5891074 décrit un pansement compressif comprenant une mousse de polymère absorbante placée en regard du point de ponction ou d'infusion. La mousse de polymère absorbante est par exemple une mousse de polyuréthane commercialisée par la société Avitar sous la marque Hydrasorb®. En variante, une pièce en acier à ressort ou en matériau polymère tel que polycarbonate, polyéthylène, polyuréthane est placée en contact direct avec la peau. Le pansement décrit dans le document US 5891074, comme un très grand nombre de pansements proposés dans l'art antérieur, vise l'application automatique d'une pression au lieu de ponction ou d'infusion, en lieu et place d'une application manuelle.

Le document US 2006/0155235 décrit un pansement compressif hémostatique. Lors de la pose du pansement, un film protecteur est enlevé permettant la libération d'une poudre hémostatique sur la peau du patient. L'agent hémostatique en poudre est par exemple un chitosane.

Au-delà des agents hémostatiques cités dans le document US 2006/0155235, on connaît dans l'art antérieur un grand nombre d'agents chimiques favorisant l'hémostase (à base de gélatine, de collagène, d'oxyde de cellulose, de chitosane). Bachtell et al (Treatment of dialysis access puncture wound bleeding with chitosan dressings, Dialysis & Transplantation, Novembre 2006*)* décrivent l'utilisation d'un pansement hémostatique commercialisé par la société HemCon, ce pansement permettant de réduire à quelques minutes le temps de compression sur les FAV pour obtenir l'hémostase. Les pansements proposés par la société HenCon ont été largement utilisés dans des contextes d'urgence. Ces pansements, par exemple commercialisés sous l'appellation Hencon Chito-Flex ® contiennent des agents hémostatiques (dérivé de chitosane, voir http://www.fda.gov). Ces pansements présentent l'inconvénient d'occulter le point de ponction que l'on ne peut surveiller visuellement tout en comprimant le point de saignement cutané.

L'invention vise à proposer un dispositif et un procédé permettant de panser un point de ponction ou d'infusion, ce dispositif ne présentant pas les inconvénients de ceux connus antérieurement, et assurant une protection élevée contre les AES, les hémorragies et les contaminations bactériennes, en particulier mais non exclusivement pour les abords vasculaires de dialyse.

A ces fins, l'invention se rapporte, selon un premier aspect, à un pansement pour point de ponction ou d'infusion, comprenant une première partie adhésive et une deuxième partie pourvue d'une compresse, la première partie étant adhésive sur une de ses faces, ce pansement comprenant une troisième partie adhésive sur une de ses faces et apte à venir recouvrir la première partie, la deuxième partie étant alors placée entre la première partie et la troisième partie, la première partie étant destinée à être fixée sur la peau au point de ponction ou d'infusion.

Avantageusement, la première partie est formée d'un matériau transparent, translucide ou semi-transparent.

Avantageusement, la première partie est réalisée en un matériau semi perméable, par exemple micro-perforé.

Dans certaines mises en oeuvre, la première partie est formée d'une bande de polyéthylène pourvue d'un adhésif acrylique, notamment pourvue de perforations d'un diamètre inférieur à 0.5 mm, la densité de perforations étant de l'ordre de cent par centimètre carré.

Dans certaines mises en oeuvre, la première partie s'étend longitudinalement, la deuxième partie étant articulée suivant une zone d'articulation longitudinale ou transversale à la première partie.

Dans certaines mises en oeuvre, la troisième partie et la première partie s'étendent sensiblement longitudinalement et sont articulées l'une à l'autre, la première partie et la troisième partie étant articulées longitudinalement ou transversalement l'une à l'autre.

Avantageusement, la troisième partie est de longueur ou de surface sensiblement égale ou supérieure à celle de la première partie.

Avantageusement, au moins la première partie ou la troisième partie est réalisée en un matériau élastique, en particulier suivant les deux directions longitudinale et/ou transversale.

Dans certaines mises en oeuvre, la troisième partie est de forme sensiblement identique à la première partie, par exemple ovale, carrée ou rectangulaire.

L'invention se rapporte, selon un deuxième aspect, à un kit de perfusion, de drainage, ou de cathétérisme, comprenant un pansement tel que présenté ci-dessus, et une aiguille ou une canule ou un cathéter ou un drain.

L'invention se rapporte, selon un troisième aspect, à l'utilisation, en tant que pansement de fistule artério-veineuse FAV, d'un multicouche comprenant :
- une première bande adhésive transparente micro-perforée ;
- un matériau absorbant ;
- une deuxième bande adhésive, articulée à la première bande adhésive.

Avantageusement, dans une première étape, la première bande adhésive est placée au point souhaité. Dans une deuxième étape, le matériau absorbant est appliqué avec pression contre la première bande adhésive. Dans une troisième étape, l'aiguille de ponction veineuse ou artérielle est complètement retirée, la pression étant maintenue sur le matériau absorbant. Dans une quatrième étape, le matériau absorbant est écarté de la première bande adhésive, permettant un contrôle visuel de l'hémostase. Dans une cinquième étape, la deuxième bande adhésive est rapportée au dessus du matériau absorbant.

D'autres objets et avantages de l'invention apparaîtront à la lumière de la description suivante de modes de réalisation, description qui va être effectuée en se référant aux dessins annexés, dans lesquels :
- la figure 1 est une vue en perspective d'un avant bras de patient, pourvu d'une FAV, une aiguille à ailettes étant en place dans cette FAV ;
- la figure 2 est une vue analogue à la figure 1, montrant une première étape du procédé ;
- la figure 3 est une vue analogue aux figures 1 et 2, montrant une deuxième étape du procédé ;
- la figure 4 est une vue analogue aux figures 1 à 3, montrant une troisième étape du procédé ;
- la figure 5 est une vue analogue aux figures 1 à 4, montrant une quatrième étape du procédé ;
- la figure 6 est une vue analogue aux figures 1 à 5, montrant une cinquième étape du procédé ;
- la figure 7 est une vue en plan d'un pansement, selon un mode de réalisation ;
- la figure 8 est une vue en perspective du pansement représenté en figure 7.
- la figure 9 est une vue en plan d'un pansement, selon une variante de réalisation ;
- la figure 10 est une vue de dessus du pansement de la figure 9, dans une étape intermédiaire de mise en place ;
- la figure 11 est une vue de dessus du pansement de la figure 9, dans une position de pose ;
- la figure 12 est une vue en plan d'une première face d'un pansement selon un autre mode de réalisation ;
- la figure 13 est une vue en plan de la seconde face du pansement représenté en figure 12 ;
- la figure 14 est une représentation schématique de la pose du pansement représenté en figures 12 et 13, sur l'avant bras d'un patient.

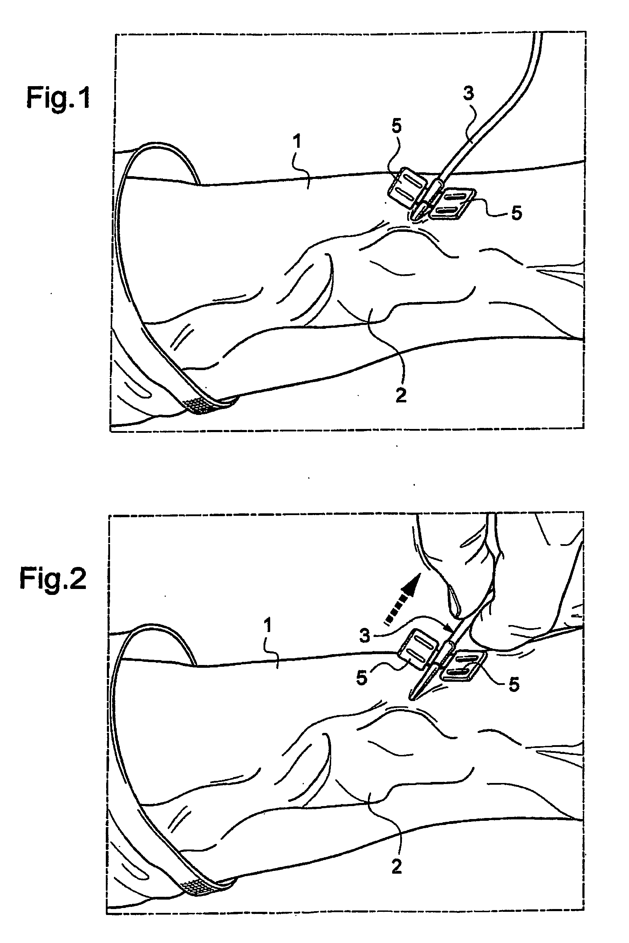

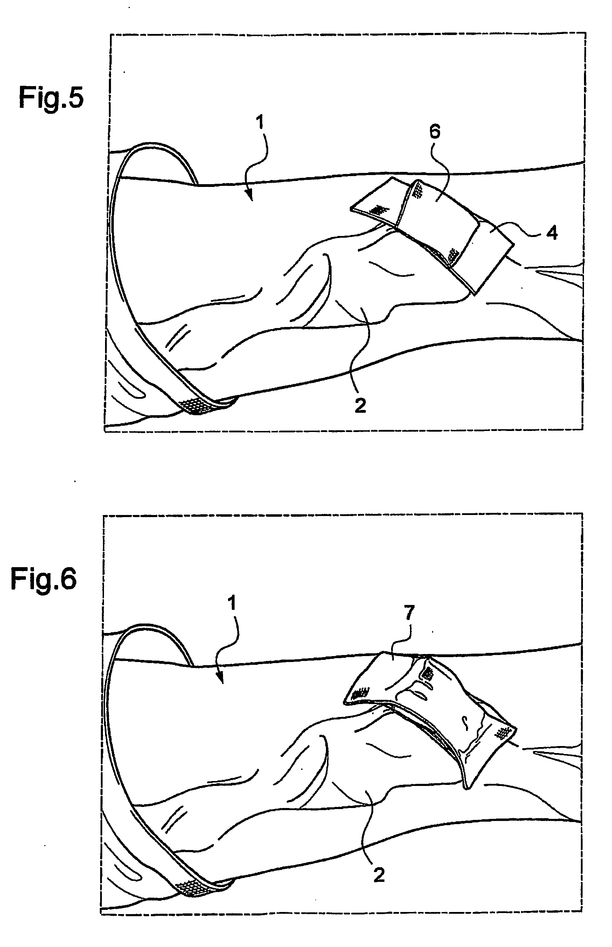

On se reporte aux figures 1 à 7.

Sur la figure 1 est représenté l'avant bras 1 d'un patient, porteur d'une FAV. A fins de simplification, une seule aiguille est encore en place dans cette FAV. Ainsi que le comprendra l'homme du métier, la procédure décrite en référence aux figures s'applique successivement aussi bien à l'aiguille de la voie veineuse qu'à l'aiguille de la voie artérielle.

Sur les figures annexées, l'aiguille est du type aiguille à ailettes. Il est entendu toutefois que la procédure décrite concerne également les aiguilles dépourvues d'ailettes, ou bien encore les cathéters (par exemple de type cathlon®) ou bien encore les canules.

La FAV, abord vasculaire de première indication et le plus répandu pour le patient dialysé, entraîne l'apparition de zones anévrismales 2, visibles sur les figures, la peau étant très déformée au voisinage de la FAV. Les FAV sont souvent de petite longueur, de sorte que les deux aiguilles artère et veine doivent être placées proches l'une de l'autre. L'utilisation de bandes de fixation peut provoquer une irritation répétée de la peau, favorisant les excoriations particulièrement pourvoyeuses de contamination bactérienne. Les fistules peuvent être maintenues actives pendant de très nombreuses années, et la peau de certains dialysés est peu souple, mince et fragile du fait de l'âge.

Dans une première étape, représentée en figure 2, l'aiguille 3 est légèrement retirée.

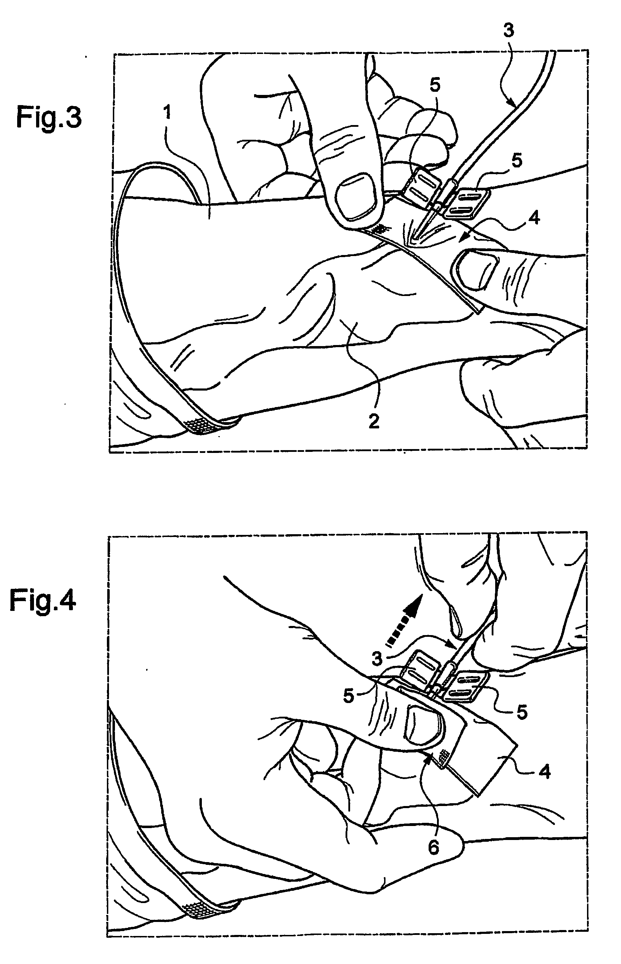

Dans une deuxième étape, représentée en figure 3, une bande adhésive 4 est placée au point de ponction. Cette bande adhésive 4 est transparente, semi-transparente ou translucide. Avantageusement, cette bande adhésive 4 est perméable aux liquides, par exemple microperforée. Avantageusement, cette bande adhésive 4 est porteuse de composés hémostatiques, par exemple mélangés à l'adhésif. Il est à noter que la bande adhésive 4 ne vient pas coller l'aiguille contre la peau du patient, les ailettes 5 n'étant en particulier pas recouvertes par la bande adhésive 4.

Dans une troisième étape, une compresse 6 est appliquée avec pression contre la bande adhésive, au droit du point de ponction et l'aiguille 3 est complètement retirée, la pression étant maintenue sur la compresse 6. Il est à noter que la bande adhésive est placée entre la compresse et la peau du patient.

Dans une quatrième étape, la pression sur la compresse 6 est relâchée et un contrôle visuel de l'hémostase peut est effectué. Il est à noter que la bande adhésive n'a pas à être enlevée, de sorte que le point de ponction reste recouvert lors du contrôle visuel de l'hémostase. Les risques d'AES sont ainsi supprimés, de même que les risques de saignement liés à un enlèvement du clou plaquettaire.

Dans une cinquième étape, une bande adhésive 7 est rapportée au dessus de la compresse 6.

La mise en oeuvre de ce procédé permet de réduire fortement le temps de compression aux points de ponction des FAV.

Il résulte de l'expérience du demandeur que les mécanismes conduisant à cette très importante réduction du temps de compression pourraient être les suivants.

Dans une première phase, après mise en place de la bande adhésive recouvrant le point de ponction, le sang sortant du point de ponction ne s'écoule pas sous la bande adhésive 4 et reste *confiné.* Avant son retrait, l'aiguille occupe un espace entre la peau et la bande adhésive 4, cet espace pouvant former un court chemin de passage au sang sortant du point de ponction. Cette phase de confinement est d'une durée variable en fonction de l'état d'hydratation du sang total : plus le patient est en hyper hydratation, plus cette première phase est longue. La durée de cette première phase est augmentée par la prise d'anti vitamine K ou en présence d'anomalie plaquettaire. La durée de cette première phase est également augmentée lorsque la FAV est le siège d'une sténose ou lorsque la ponction de la FAV est réalisée sur une zone anévrysmale ou proche de l'anastomose. La durée de cette première phase peut être réduite en exerçant une pression douce sur l'anastomose de deux minutes environ.

Dans une deuxième phase, plus courte que la première, le sang est *tamisé* par son passage au travers de la bande adhésive 4, perméable au liquide, et le sérum est *absorbé* par la compresse 6. La concentration en éléments figurés augmente dans le sang présent au point de ponction, du fait de ce tamisage et de cette absorption du sérum. La viscosité du sang présent au point de ponction augmente. Les perforations de la bande adhésive 4 sont progressivement obstruées par du sang visqueux.

La mise en oeuvre du procédé permet également de réduire fortement les risques de reprise de saignement.

Il résulte de l'expérience du demandeur que les mécanismes conduisant à cette réduction du risque de reprise de saignement pourraient être les suivants.

Le confinement et le tamisage du sang s'écoulant du point de ponction conduit à la création de croutes de petites étendues. Lors du retrait de la compresse 6, le risque de décollement de ces croûtes est ainsi réduit.

La bande adhésive 4 semi perméable transparente stérile protège le site de ponction vasculaire et le laisse visible à tout moment sans risque de projection de sang ni de collage direct de la compresse 6 sur le point de ponction.

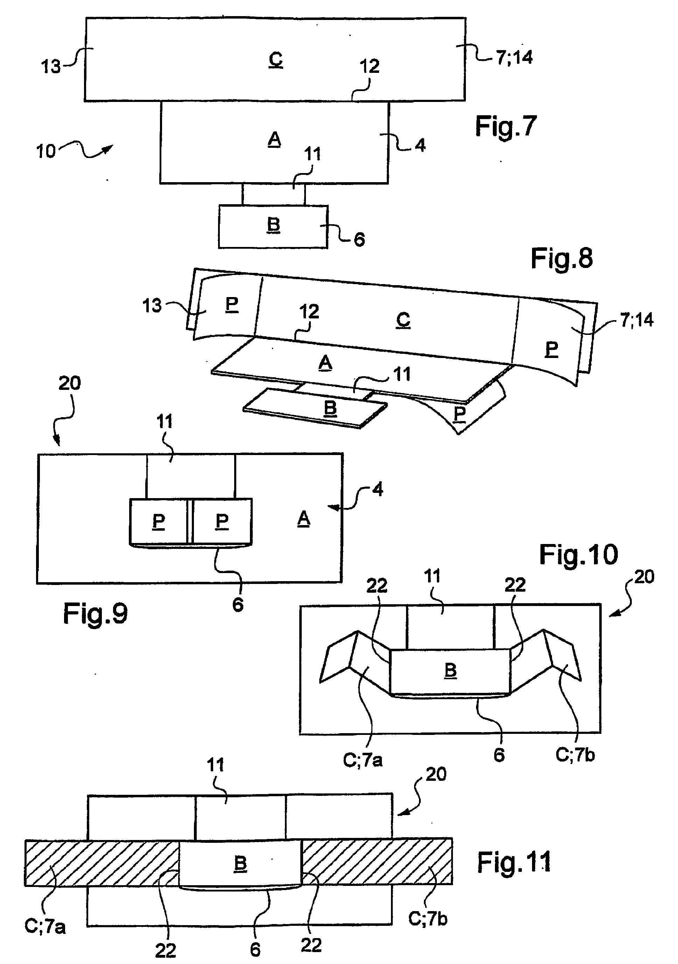

On se reporte maintenant aux figures 7 et 8 illustrant un mode de réalisation d'un pansement, permettant l'emploi du procédé décrit en référence aux figures 1 à 6.

Le pansement 10 représentée en figures 7 et 8 comprend une première partie A formant ladite bande adhésive 4. Avantageusement, une feuille pelable vient recouvrir la face adhésive de cette première partie A.

Le pansement 10 comprend, articulé à cette première partie A, une deuxième partie B formant ladite compresse 6. Dans le mode de réalisation représenté, la deuxième partie B est reliée à la première partie A par une languette 11. Dans une mise en oeuvre, cette languette 11 est pourvue d'une ligne d'affaiblissement permettant de séparer la deuxième partie B de la première partie A du pansement 10. Par ligne d'affaiblissement, on désigne ici toute ligne de rainage, rainurage, perforations, prédécoupe, amincissement, la technique mise en oeuvre pour réaliser cette ligne d'affaiblissement étant fonction, ainsi qu'il est connu en soit, du type de matériau employé pour le pansement. Par ligne d'affaiblissement, on désigne également toute trace sur le pansement guidant l'opérateur pour la découpe du pansement à l'aide d'un outil.

Le pansement 10 comprend, articulé à la première partie A, une troisième partie C formant ladite bande adhésive 7. Avantageusement, une feuille pelable P vient recouvrir, avant usage, la face adhésive de la troisième partie C. Dans la réalisation représentée, la troisième partie C est articulée à la première partie A par une ligne d'articulation 12 sensiblement longitudinale, c'est-à-dire s'étendant suivant la plus grande dimension de la première partie A. Dans une mise en oeuvre, cette ligne d'articulation 12 est une ligne d'affaiblissement au sens défini ci-dessus, permettant la séparation de la troisième partie C de la première partie A du pansement 10.

Dans une mise en oeuvre, la compresse 6 est au moins en partie surmontée d'une plaque semi-rigide et transparente, par exemple en polycarbonate ou PET. Cette plaque participe à la compression du point de ponction, lorsque la bande adhésive 7 est placée sous tension contre la compresse 6.

Avantageusement, la bande adhésive 4 formée par la première partie A du pansement est semi-perméable et autorise la diffusion d'au moins certains composés du sang du point de ponction vers la compresse 6.

Dans un mode de mise en oeuvre avantageux, le pansement, à l'exception de la compresse 6, est réalisé en matériau polymère souple ou tissu enduit. Le pansement, à l'exception de la compresse, est avantageusement entièrement transparent, translucide ou semi transparent.

La souplesse du pansement permet de suivre les contours de la peau, y compris au voisinage des zones anévrismales des FAV. Avantageusement, le matériau constituant les parties latérales 13, 14 de la troisième partie C et le cas échéant tout le pansement 1, est extensible suivant au moins une direction longitudinale et, encore plus avantageusement, suivant les deux directions longitudinale et transversale. Le pansement est ainsi encore plus adaptable aux différentes courbures du corps du patient.

On se reporte maintenant aux figures 9 à 11.

Le pansement 20 représenté en figures 9 à 11 comprend une première partie A formant ladite bande adhésive 4. Avantageusement, une feuille pelable vient recouvrir la face adhésive de cette première partie A.

Le pansement 20 comprend, articulé à cette première partie A, une deuxième partie B formant ladite compresse 6. Dans le mode de réalisation représenté, la deuxième partie B est reliée à la première partie A par une languette 11. Dans une mise en oeuvre, cette languette 11 est pourvue d'une ligne d'affaiblissement permettant de séparer la deuxième partie B de la première partie A du pansement 20. Par ligne d'affaiblissement, on désigne ici toute ligne de rainage, rainurage, perforations, prédécoupe, amincissement, la technique mise en oeuvre pour réaliser cette ligne d'affaiblissement étant fonction, ainsi qu'il est connu en soit, du type de matériau employé pour le pansement. Par ligne d'affaiblissement, on désigne également toute trace sur le pansement guidant l'opérateur pour la découpe du pansement à l'aide d'un outil.

Le pansement 20 comprend, articulé à la première partie A, une troisième partie C formant ladite bande adhésive 7. Avantageusement, une feuille pelable P vient recouvrir, avant usage, la face adhésive de la troisième partie C.

Dans la réalisation représentée, la troisième partie C forme deux pattes 7a, 7b articulées à la deuxième partie B par des lignes d'articulation 22 sensiblement transversales, c'est-à-dire s'étendant suivant la plus petite dimension de la première partie A. Dans une mise en oeuvre, ces lignes d'articulation 22 sont des lignes d'affaiblissement au sens défini ci-dessus, permettant la séparation de la troisième partie C de la première partie A du pansement 20.

Dans une mise en oeuvre, la compresse 6 est au moins en partie surmontée d'une plaque semi-rigide et transparente, par exemple en polycarbonate ou PET. Cette plaque participe à la compression du point de ponction, lorsque les bandes adhésives 7a, 7b sont placées sous tension contre la compresse 6.

Avantageusement, la bande adhésive 4 formée par la première partie A du pansement est semi-perméable et autorise la diffusion d'au moins certains composés du sang du point de ponction vers la compresse 6.

Dans un mode de mise en oeuvre avantageux, le pansement, à l'exception de la compresse 6, est réalisé en matériau polymère souple ou tissu enduit. Le pansement, à l'exception de la compresse, est avantageusement entièrement transparent, translucide ou semi transparent.

La souplesse du pansement permet de suivre les contours de la peau, y compris au voisinage des zones anévrismales des FAV. Avantageusement, le matériau constituant les parties latérales 7a, 7b de la troisième partie C et le cas échéant tout le pansement 1, est extensible suivant au moins une direction longitudinale et, encore plus avantageusement, suivant les deux directions longitudinale et transversale. Le pansement est ainsi encore plus adaptable aux différentes courbures du corps du patient.

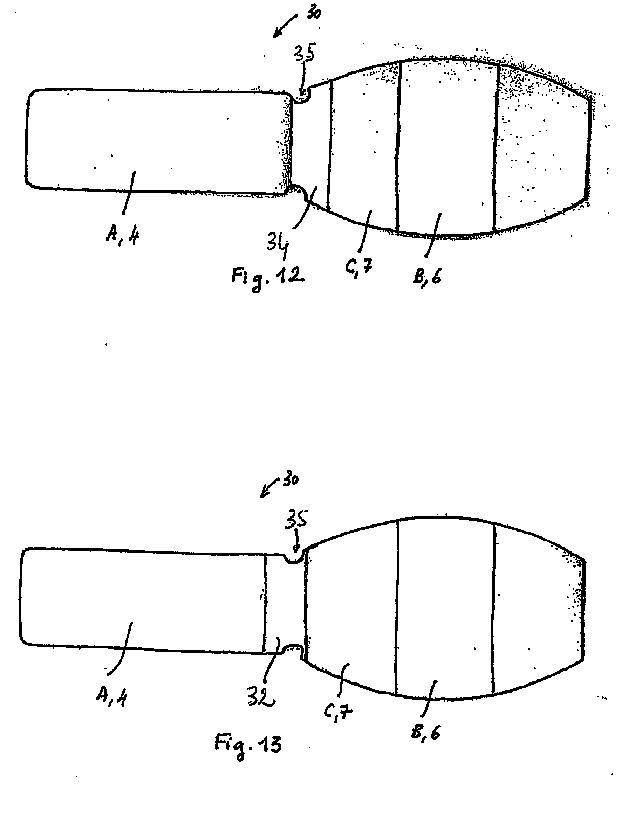

On se reporte maintenant aux figures 12 et 13.

Le pansement 30 représentée en figures 12 et 13 comprend une première partie A, 4. Avantageusement, une bande pelable 31 vient recouvrir une première face, adhésive, de cette première partie A. Une patte de préhension 32 facilite l'enlèvement de cette bande pelable 31.

Le pansement 30 comprend, articulé à cette première partie A, une troisième partie C pourvue d'un matériau absorbant B tel qu'une compresse 6. Dans le mode de réalisation représenté, la deuxième partie B est fixée sur la troisième partie C formant la bande adhésive 7.

La première partie A et l'ensemble formé par les deuxième et troisième parties B, C sont articulés autour d'une zone transversale 35. Cette zone d'articulation 35 peut comprendre une ligne d'affaiblissement telle que définie auparavant en référence aux figures 7 à 11.

La compresse B, 6 et la bande adhésive 7 sont recouvertes, avant usage, d'une bande pelable 33. Avantageusement, une patte de préhension 34 facilite l'enlèvement de cette bande pelable 33.

Dans une mise en oeuvre, la compresse B, 6 est au moins en partie surmontée d'une plaque semi-rigide et transparente, par exemple en polycarbonate ou PET. Cette plaque participe à la compression du point de ponction ou d'infusion Pl, lorsque la bande adhésive 7 est placée sous tension contre la compresse B, 6.

Avantageusement, la première partie A du pansement 30 est semi-perméable et autorise la diffusion d'au moins certains composés du sang du point de ponction ou d'infusion, vers la compresse B, 6.

Dans un mode de mise en oeuvre avantageux, le pansement, à l'exception de la compresse B, 6, est réalisé en matériau polymère souple ou tissu enduit. Le pansement, à l'exception de la compresse B, 6, est avantageusement entièrement transparent, translucide ou semi transparent.

La souplesse du pansement permet de suivre les contours de la peau, y compris au voisinage des zones anévrismales des FAV. Avantageusement, le matériau constituant la troisième partie C et le cas échéant tout le pansement 30, est extensible suivant au moins une direction longitudinale et, encore plus avantageusement, suivant les deux directions longitudinale et transversale. Le pansement est ainsi encore plus adaptable aux différentes courbures du corps du patient.

La troisième partie C est avantageusement de surface supérieure à celle de la première partie A et est ainsi apte à la recouvrir complètement.

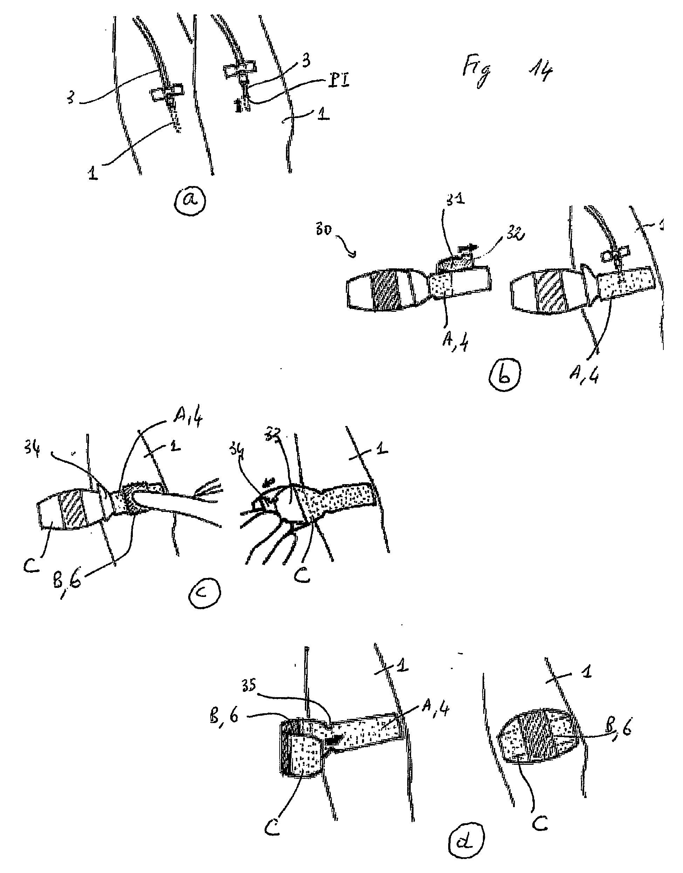

On se rapporte maintenant à la figure 14.

Sur la figure 14 est représenté l'avant bras 1 d'un patient, une aiguille 3 étant en place dans cet avant bras, à un point de ponction ou d'infusion P, par exemple dans une FAV.

L'aiguille 3 représentée est du type aiguille à ailettes. Il est entendu toutefois que la procédure qui va être décrite concerne également les aiguilles dépourvues d'ailettes, ou bien encore les cathéters (par exemple de type cathlon®) ou bien encore les canules.

Dans une première étape, l'aiguille 1 est avantageusement légèrement retirée, de manière analogue à ce qui a été décrit en référence à la figure 2. Ce léger retrait de l'aiguille 1 est symbolisé par une flèche en a) de la figure 14.

La bande pelable 31 recouvrant la première partie A, 4 du pansement 30 est enlevée manuellement, cette opération étant facilitée par la présence de la patte de préhension 32.

Après enlèvement de la bande pelable 31, une première face de la première partie A, 4 du pansement 30 est placée au point souhaité, en recouvrant le point de ponction ou d'infusion Pl, ainsi qu'il apparaît en b) de la figure 14. Cette première face de la première partie A, 4 du pansement est adhésive.

Puis, un matériau absorbant tel qu'une compresse 6 est appliquée avec pression contre la seconde face de la première partie A, 4 du pansement 30, au droit du ponction de ponction ou d'infusion Pl. Avantageusement, la seconde face de la première partie du pansement est non adhésive.

Le matériau absorbant peut être enlevé à l'issue d'un temps court, ainsi qu'il a été exposé précédemment, de sorte à contrôler l'arrêt de saignement au point de ponction ou d'infusion Pl.

Puis, la bande pelable 33 recouvrant la face supérieure de la troisième partie C du pansement 30 est enlevée manuellement, cette opération étant facilitée par la présence de la patte de préhension 34.

Après enlèvement de la bande pelable 33, une première face de la troisième partie C du pansement est repliée autour de la zone d'articulation 35 de sorte à venir recouvrir la première partie A, 4 du pansement. La première face de la troisième partie C du pansement est adhésive. Cette première face est avantageusement pourvue d'un matériau absorbant tel qu'une compresse B, 6.

Lorsque la troisième partie C est repliée contre la première partie A, 4 du pansement 30, le matériau absorbant B, 6 est placé, au droit du point de ponction ou d'infusion Pl, entre la première partie A et la troisième partie C du pansement 30.

Avantageusement, la seconde face de la troisième partie C du pansement est non adhésive.

Avantageusement, la première partie et la troisième partie du pansement comprennent une bande adhésive 4 perméable microperforée.

La perforation est avantageusement réalisée en mettant en oeuvre un procédé comprenant les étapes suivantes :
- dépôt de colle sur la face du film devant être rendue adhésive, par exemple un film de polyéthylène (PE), notamment un film PE basse densité de 60 microns d'épaisseur, l'adhésif étant à base acrylique, sur une épaisseur de 30 microns environ ;
- dépôt d'un liner recouvrant la face encollée ;
- perforation du film, par exemple par aiguilletage à chaud, suivant un motif à maille carrée ou losange, en une ou deux passes, la densité de trous étant avantageusement de l'ordre de 110 par centimètre carré ;
- dépose du film de protection et mise en place d'une bande pelable.

Avantageusement, la bande adhésive 4 comprend deux types de perforations :
- une première série de perforations, destinée au tamisage du sang au point de ponction, cette première série de perforations étant par exemple à motif aléatoire ou losange,
- une deuxième série de perforations, destinée à faciliter, le cas échéant, la découpe manuelle de la bande adhésive, cette deuxième série de perforations ayant un motif carré ou rectangulaire par exemple.

Avantageusement, les perforations destinées au tamisage sont des micro-perforations obtenues sans enlèvement de matière, par exemple par aiguilletage.

Avantageusement, la bande adhésive est élastique et la perforation est effectuée avec mise sous tension de la bande adhésive, de sorte que les micropores sont sensiblement fermés après relâchement de la tension de la bande. Lors de la pose du pansement, l'étirement manuel de la bande adhésive provoque l'ouverture de ces micropores.

## Revendications

1. Pansement pour point de ponction ou d'infusion, comprenant une première partie adhésive (A) et une deuxième partie (B) pourvue d'une compresse (6), la première partie (A) étant adhésive sur une de ses faces, **caractérisé en ce qu'**il comprend une troisième partie adhésive (C) sur une de ses faces et apte à venir recouvrir la première partie (A), la deuxième partie (B) étant alors placée entre la première partie (A) et la troisième partie (C), la première partie (A) étant destinée à être fixée sur la peau au point de ponction ou d'infusion.

2. Pansement selon la revendication 1, **caractérisé en ce que** la première partie (A) est formée d'un matériau transparent, translucide ou semi-transparent.

3. Pansement selon la revendication 1 ou 2, **caractérisé en ce que** la première partie (A) est réalisée en un matériau semi perméable.

4. Pansement selon la revendication 3, **caractérisé en ce que** la première partie (A) est microperforée.

5. Pansement selon la revendication 4, **caractérisé en ce que** la première partie (A) est formée d'une bande de polyéthylène pourvue d'un adhésif acrylique.

6. Pansement selon la revendication 5, **caractérisé en ce que** la première partie (A) est pourvue de perforations d'un diamètre inférieur à 0.5 mm.

7. Pansement selon la revendication 6, **caractérisé en ce que** la densité de perforations est de l'ordre de cent par centimètre carré.

8. Pansement selon l'une quelconque des revendications 1 à 7, **caractérisé en ce que** la première partie (A) s'étend longitudinalement, la deuxième partie (B) étant articulée suivant une zone d'articulation longitudinale ou transversale à la première partie (A).

9. Pansement selon l'une quelconque des revendications 1 à 8, **caractérisé en ce que** la troisième partie (C) et la première partie (A) s'étendent sensiblement longitudinalement et sont articulées l'une à l'autre.

10. Pansement selon la revendication 9, **caractérisé en ce que** la première partie (A) et la troisième partie (C) sont articulées longitudinalement ou transversalement l'une à l'autre.

11. Pansement selon la revendication 10, **caractérisé en ce que** la troisième partie (C) est de longueur sensiblement égale ou supérieure à celle de la première partie (A).

12. Pansement selon l'une quelconque des revendications précédentes, **caractérisé en ce qu'**au moins la première partie (A) ou la troisième partie (C) est réalisée en un matériau élastique, en particulier suivant les deux directions longitudinale et/ou transversale.

13. Pansement selon l'une quelconque des revendications précédentes, **caractérisé en ce que** la troisième partie (C) est de forme sensiblement identique à la première partie (A), par exemple ovale, carrée ou rectangulaire.

14. Kit de perfusion, de drainage, ou de cathétérisme, comprenant un pansement tel que présenté dans l'une quelconque des revendications précédentes et une aiguille ou une canule ou un cathéter ou un drain.

15. Utilisation, en tant que pansement de fistule artério-veineuse FAV, d'un multicouche comprenant :
- une première bande adhésive (A) transparente micro-perforée ;
- un matériau absorbant ;
- une deuxième bande adhésive (C), articulée à la première bande adhésive (A),
la première bande adhésive (A) étant destinée à être fixée sur la peau au point de ponction ou d'infusion.

16. Utilisation selon la revendication 15, **caractérisée en ce que** :
- dans une première étape, la première bande adhésive (A, 4) est placée au point souhaité;
- dans une deuxième étape, le matériau absorbant est appliqué avec pression contre la première bande adhésive (A);
- dans une troisième étape, l'aiguille de ponction veineuse ou artérielle est complètement retirée, la pression étant maintenue sur le matériau absorbant ;
- dans une quatrième étape, le matériau absorbant est écarté de la première bande adhésive (A), permettant un contrôle visuel de l'hémostase ;
- dans une cinquième étape, la deuxième bande adhésive (C) est rapportée au dessus du matériau absorbant.

## Patentansprüche

1. Verband für eine Punktions- oder Infusionsstelle, umfassend einen ersten haftenden Teil (A) und einen zweiten Teil (B), der mit einer Kompresse (6) versehen ist, wobei der erste Teil (A) auf einer seiner Seiten haftend ist, **dadurch gekennzeichnet, dass** er einen dritten auf einer seiner Seiten haftenden Teil (C) umfasst, der geeignet ist, den ersten Teil (A) zu bedecken, wobei der zweite Teil (B) nun zwischen dem ersten Teil (A) und dem dritten Teil (C) angeordnet ist, wobei der erste Teil (A) dazu bestimmt ist, auf der Haut an der Punktions- oder Infusionsstelle befestigt zu werden.

2. Verband nach Anspruch 1, **dadurch gekennzeichnet, dass** der erste Teil (A) von einem durchsichtigen, durchscheinenden oder halb-durchsichtigen Material gebildet ist.

3. Verband nach Anspruch 1 oder 2, **dadurch gekennzeichnet, dass** der erste Teil (A) aus einem halb durchlässigen Material hergestellt ist.

4. Verband nach Anspruch 3, **dadurch gekennzeichnet, dass** der erste Teil (A) mikroperforiert ist.

5. Verband nach Anspruch 4, **dadurch gekennzeichnet, dass** der erste Teil (A) von einem Polyethylenband gebildet ist, das mit einem Acrylhaftmittel versehen ist.

6. Verband nach Anspruch 5, **dadurch gekennzeichnet, dass** der erste Teil (A) mit Perforationen mit einem Durchmesser unter 0,5 mm versehen ist.

7. Verband nach Anspruch 6, **dadurch gekennzeichnet, dass** die Perforationsdichte ungefähr hundert pro Quadratzentimeter beträgt.

8. Verband nach einem der Ansprüche 1 bis 7, **dadurch gekennzeichnet, dass** sich der erste Teil (A) längs erstreckt, wobei der zweite Teil (B) entlang einer Längs- oder Quergelenkszone am ersten Teil (A) angelenkt ist.

9. Verband nach einem der Ansprüche 1 bis 8, **dadurch gekennzeichnet, dass** sich der dritte Teil (C) und der erste Teil (A) im Wesentlichen längs erstrecken und aneinander angelenkt sind.

10. Verband nach Anspruch 9, **dadurch gekennzeichnet, dass** der erste Teil (A) und der dritte Teil (C) längs oder quer aneinander angelenkt sind.

11. Verband nach Anspruch 10, **dadurch gekennzeichnet, dass** der dritte Teil (C) eine im Wesentlichen gleiche oder größere Länge als jene des ersten Teils (A) hat.

12. Verband nach einem der vorhergehenden Ansprüche, **dadurch gekennzeichnet, dass** mindestens der erste Teil (A) oder der dritte Teil (C) aus einem elastischen Material insbesondere entlang der beiden Längs- und/oder Querrichtungen hergestellt ist.

13. Verband nach einem der vorhergehenden Ansprüche, **dadurch gekennzeichnet, dass** der dritte Teil (C) eine im Wesentlichen identische Form wie der erste Teil (A), beispielsweise oval, quadratisch oder rechteckig, hat.

14. Perfusions-, Drainage- oder Katheterlegesatz, umfassend einen Verband, wie in einem der vorhergehenden Ansprüche dargestellt, und eine Nadel oder Kanüle oder einen Katheter oder Drain.

15. Verwendung einer mehrschichtigen Vorrichtung als Verband einer arteriovenösen Fistel FAV, umfassend:
- ein erstes mikroperforiertes durchsichtiges haftendes Band (A);
- ein absorbierendes Material;
- ein zweites haftendes Band (C), das am ersten haftenden Band (A) angelenkt ist,
wobei das erste haftende Band (A) dazu bestimmt ist, auf der Haut am Punktions- oder Infusionspunkt befestigt zu werden.

16. Verwendung nach Anspruch 15, **dadurch gekennzeichnet, dass**:
- in einem ersten Schritt das erste haftende Band (A, 4) am gewünschten Punkt angeordnet wird;
- in einem zweiten Schritt das absorbierende Material mit Druck am ersten haftenden Band (A) angeordnet wird;
- in einem dritten Schritt die Venen- oder Arterienpunktionsnadel zur Gänze herausgezogen wird, wobei der Druck auf dem absorbierenden Material aufrechterhalten wird;
- in einem vierten Schritt das absorbierende Material vom ersten haftenden Band (A) entfernt wird, wodurch eine Sichtkontrolle der Hämostase möglich ist;
- in einem fünften Schritt das zweite haftende Band (C) über dem absorbierenden Material angebracht wird.

## Claims

1. Dressing for a puncture site or infusion site, comprising a first adhesive part (A) and a second part (B) provided with a compress (6), the first part (A) being adhesive on one of its faces, **characterized in that** it comprises a third adhesive part (C) on one of its faces that is capable of covering the first part (A), the second part (B) then being placed between the first part (A) and the third part (C), the first part (A) being intended to be attached to the skin at the puncture site or infusion site.

2. Dressing according to Claim 1, **characterized in that** the first part (A) is formed from a transparent, translucent or semi-transparent material.

3. Dressing according to Claim 1 or 2, **characterized in that** the first part (A) is made from a semi-permeable material.

4. Dressing according to Claim 3, **characterized in that** the first part (A) is micro-perforated.

5. Dressing according to Claim 4, **characterized in that** the first part (A) is formed from a strip of polyethylene provided with an acrylic adhesive.

6. Dressing according to Claim 5, **characterized in that** the first part (A) is provided with perforations having a diameter of less than 0.5 mm.

7. Dressing according to Claim 6, **characterized in that** the density of perforations is of the order of 100 per square centimetre.

8. Dressing according to any one of Claims 1 to 7, **characterized in that** the first part (A) extends longitudinally, the second part (B) being articulated along an articulation zone longitudinal or transverse to the first part (A).

9. Dressing according to any one of Claims 1 to 8, **characterized in that** the third part (C) and the first part (A) extend substantially longitudinally and are articulated to one another.

10. Dressing according to Claim 9, **characterized in that** the first part (A) and the third part (C) are articulated longitudinally or transversely to one another.

11. Dressing according to Claim 10, **characterized in that** the third part (C) has a length substantially equal to or greater than that of the first part (A).

12. Dressing according to any one of the preceding claims, **characterized in that** at least the first part (A) or the third part (C) is made from a material that is elastic, in particular along the two longitudinal and/or transverse directions.

13. Dressing according to any one of the preceding claims, **characterized in that** the third part (C) is of substantially identical shape to the first part (A), for example oval, square or rectangular.

14. Infusion, drain or catheterization kit comprising a dressing as presented in any one of the preceding claims and a needle or cannula or catheter or drain.

15. Use, as an arteriovenous fistula AVF dressing, of a multilayer comprising:
- a first adhesive strip (A) that is transparent and micro-perforated;
- an absorbent material;
- a second adhesive strip (C) that is articulated to the first adhesive strip (A),
the first adhesive strip (A) being intended to be attached to the skin at the puncture site or infusion site.

16. Use according to Claim 15, **characterized in that**:
- in a first step, the first adhesive strip (A, 4) is placed at the desired site;
- in a second step, the absorbent material is applied with pressure against the first adhesive strip (A);
- in a third step, the vein or artery puncture needle is completely withdrawn, the pressure being maintained on the absorbent material;
- in a fourth step, the absorbent material is moved away from the first adhesive strip (A), allowing visual control of the haemostasis;
- in a fifth step, the second adhesive strip (C) is added on top of the absorbent material.
